Europäisches Patentamt

European Patent Office

Office européen des brevets

(11) Veröffentlichungsnummer: **0 000 124**
**B1**

(12) **EUROPÄISCHE PATENTSCHRIFT**

(45) Veröffentlichungstag der Patentschrift: **30.12.81**

(21) Anmeldenummer: **78100113.6**

(22) Anmeldetag: **07.06.78**

(51) Int. Cl.³: **A 61 K 35/16,**
**A 61 K 37/64**

(54) Mittel zur Senkung des Blutzuckerspiegels und Verbesserung der Durchblutung und Wundheilung.

(30) Priorität: **28.06.77 DE 2729096**

(43) Veröffentlichungstag der Anmeldung:
**10.01.79 Patentblatt 79/1**

(45) Bekanntmachung des Hinweises auf die Patenterteilung:
**30.12.81 Patentblatt 81/52**

(84) Benannte Vertragsstaaten:
**CH FR GB NL SE**

(56) Entgegenhaltungen:
**US - A - 3 947 575**

**CHEMICAL ABSTRACTS, vol. 79, 27505m (1973)**
**& Verh. Deut. Ges. Inn. Med. 78, 1272—5 (1972)**

**THE MERCK INDEX, 8th edition, Merck & Co.**
**Rahway, N.Y. (1968)**

**UNLISTED DRUGS, vol. 24, no. 11, 176 (1972)**

(73) Patentinhaber: **Hormon-Chemie München GmbH**
**Freisinger Landstrasse 74**
**D-8000 München 45 (DE)**

(72) Erfinder:
**Die Erfinder haben auf ihre Nennung verzichtet**

(74) Vertreter: **Allgeuer, Dorothea et al,**
**Chemie Linz AG Patentabteilung St. Peter-Strasse**
**25**
**A-4020 Linz (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 0 000 124

## Mittel zur Senkung des Blutzuckerspiegels und Verbesserung der Durchblutung und Wundheilung

Es ist bekannt, daß bestimmte niedermolekulare Peptide, die aus dem Gift der Schlange Bothrops jararaca isoliert wurden, eine blutdrucksenkende Wirkung besitzen. So ist in The Lancet 1973, S. 72, beschrieben, daß das als Pentapeptid erkannte Oligopeptid der Bezeichnung $B.P.P._{5a}$ ebenso wie das Nonapeptid der Bezeichnung $B.P.P._{9a}$ oder SQ 20881, das die Aminosäurefolge Pyroglu-Tryp-Pro-Arg-Pro-Glu-Ileu-Pro-Pro aufweist, eine starke blutdrucksenkende Wirkung bei dem durch Angiotensin I ausgelösten Hochdruck beim Menschen zeigen. Die Wirkung kann mit einer Blockierung des Enzyms erklärt werden, das im Organismus das Angiotensin I in das blutdruckwirksame Angiotensin II umwandelt. Außerdem wirkt es hemmend auf den hypertensiven Effekt des Angiotensin I bei pharmakologischen Untersuchungen mit Ratten und Hunden (vgl. US-PS 3 947 575). Die Inhibitorpeptide des Angiotensin-Umwandlungsenzyms (angiotensin-converting enzyme) wurden auch bereits mit Erfolg bei Hochdruckpatienten angewendet, vgl. The New English Journal of Medicine, 1974, S. 817—821.

Es wurde überraschenderweise festgestellt, daß diese Inhibitorpeptide eine blutzuckersenkende Wirkung aufweisen und in Kombination mit einem eiweißfreien Kälberblut extrakt, wobei die Menge 0,1 $\mu$g — 250 $\mu$g je mg Kälberblutextrakt betragen soll, auch eine Verbesserung der Durchblutung und Wundheilung bewirken. Die näheren Untersuchungen dieses Effekts haben erkennen lassen, daß diese Wirkung auf einer Verbesserung der Diffusion der Glucose durch die Zellmembranen der Nerven- und Muskelzellen beruhen dürfte. Die neue Erkenntnis der blutzuckersenkenden bzw. durchblutungsfördernden Wirkung der Inhibitorpeptide ist deshalb so überraschend, weil einerseits die bekannten Arzneimittel gegen Diabetes, sei es nun das parenteral zu applizierende Insulin oder die oralen Antidiabetika, wie z.B. die Benzolsulfonylharnstoff-Derivate, keinerlei blutdrucksenkende Wirkung erkennen lassen, während andererseits auch die bekannten blutdrucksenkenden Wirkstoffe keinen Einfluß auf den Blutzucker zeigen. Auch ist nicht bekannt, daß das System Angiotensin I — Angiotensin II in irgendeinem unmittelbaren Zusammenhang mit der Glucoseverwertung steht.

Der Zusatz dieser Inhibitorpeptide zu dem eiweißfreien Kälberblutextrakt führt zu einer Verbesserung der mit diesem Extrakt erzielten Heilungen, d.h. die Wirkung des Extraktes wird duch den Gehalt an Inhibitorpeptiden deutlich potenziert, was darauf zurückgeführt werden kann, daß der Stoffwechsel mangelhaft versorgter Gewebspartien durch die Inhibitorpeptide verbessert wird. Diese Extrakte, die bei Durchblutungs- und Stoffwechselstörungen Anwendung finden können, vgl. Med. Welt, Bd. 19, S. 198 (1968), sind u.a. unter der geschützten Bezeichnung "Actihaemyl" der Firma Solko und unter dem Namen "Actovegin" der Firma Hormonchemie im Handel. Sie können oral, intravenös oder intraarteriell infundiert oder intramuskular injiziert oder als Salbe appliziert werden. Durch die Inhibitorpeptide wird nunmehr erreicht, daß speziell bei diabetischer Stoffwechsellage die Durchblutungswirkung verstärkt wird und bei lokaler Anwendung die Wundheilung schneller eintritt.

Da die Wirkung der Inhibitorpeptide bei den erfindungsgemäßen Mitteln bereits bei Dosierungen eintritt, bei denen die Wirkung auf den Blutdruck sich noch nicht deutlich zeigt, wird hierdurch der Kreislauf nicht gestört und belastet. Die neuen Präparate stellen daher eine wertvolle Bereicherung des Arzneimittelschatzes dar.

### Beispiel 1

400 mg Blutextrakt (Extr. sanguin. deprot. sicc.) und 150 mg Inhibitorpeptid werden mit einer Zelluloseglucolat-Propylenglykol-Geleegrundlage ad 100 ml vermischt. Der erhaltene Gelee dient zur äußerlichen Anwendung bei offenen Hautdefekten.

### Beispiel 2

100 mg Blutextrakt (Extr. sanguin, deprot. sicc.) und 75 mg Inhibitorpeptid werden mit einer Polyäthylen-glycolcetylalkohol-Grundlage ad 100 ml vermischt. Die erhaltene Creme dient zur äußerlichen Anwendung bei nicht nässenden Hautschäden.

### Beispiel 3

0,5 g Blutextrakt (Extr. sanguin, deprot, sicc.) und 50 mg Inhibitorpeptid werden in aqua pro injectione ad 250 ml gelöst. Die Lösung dient zur intravenösen Infusion bei einer Infusionszeit von ca. 30 Minuten.

### Beispiel 4

100 mg Blutextrakt (Extr. sanguin, deprot. sicc.) 0,230 g NaC und 10 mg Inhibitorpeptid werden in aqua pro injectione ad 100 ml gelöst. Die erhaltene Lösung dient zur intraarteriellen Infusion.

## Patentanspruch

Mittel zur Senkung des Blutzuckerspiegels und Verbesserung der Durchblutung und Wundheilung dadurch gekennzeichnet, daß sie die Inhibitorpeptide des Angiotensin-Umwandlungsenzyms in Kombination mit einem eiweißfreien Kälberblutextrakt, der gegen Durchblutungs — und Stoffwechselstörungen wirkt, in einer Menge von 0,1 $\mu$g — 250 $\mu$g je mg Kälberblutextrakt enthalten.

**0 000 124**

**Revendication**

Agents pour abaisser la glycémie et améliorer l'irrigation sanguine et la cicatrisation des blessures, caractérisés en ce qu'ils contiennent les peptides inhibiteurs du système de transformation de l'angiotensine en combinaison avec un extrait de sang de veaux déprotéiné, agissant contre les désordres de la circulation et du métabolisme, en une quantité de 0,1 microgramme à 250 microgrammes par mg d'extrait de sang de veaux.

**Claim**

An agent for lowering the level of blood sugar and promoting the blood circulation and enhancing the healing of a wound characterized in that the said agent contains the inhibitor peptides of the angiotensin conversion enzym in combination with an extract of calf blood free from protein, which is effective against disorders of blood circulation and metabolism in an amount of 0.1 $\mu$g to 250 $\mu$g per each mg of calf blood extract.

3